# EUROPÄISCHE PATENTSCHRIFT

(11) **EP 0 685 206 B1**
(45) Veröffentlichungstag und Bekanntmachung des Hinweises auf die Patenterteilung: **06.09.2000**
(21) Anmeldenummer: 95106751.1
(22) Anmeldetag: 04.05.1995
(51) Int. Cl.: A61B 17/60

(54) **Fixateur für Fingerknochen**
Finger bone fixator
Fixateur pour os des doigts

(30) Priorität: 26.05.1994 DE 9408668 U
(43) Veröffentlichungstag der Anmeldung: 06.12.1995
(73) Patentinhaber: Waldemar Link (GmbH & Co.), 22339 Hamburg (DE)
(72) Erfinder: Ellis, M.L., Dr., NL-6566 DG Millingen a/d Rijn (NL)
(74) Vertreter: Glawe, Delfs, Moll & Partner

(56) Entgegenhaltungen:
- EP-A- 0 377 744
- WO-A-86/02821
- WO-A-88/05288
- WO-A-92/15258

## Beschreibung

Bekannte externe Fixateure, wie sie auch zur Behandlung von Frakturen im Hand- und Fingerbereich verwendet werden, sind schwer und ausladend, was nicht nur hinderlich ist, sondern auch ihre Anwendung in manchen Fällen ausschließt. Sie sind auch kostspielig, weil sie auf der Verwendung von Spezialteilen beruhen.

Der Erfindung liegt die Aufgabe zugrunde, einen vielseitig verwendbaren, anpassungsfähigen, leichten und kostengünstigen Fixateur, insbesondere für Frakturen im Hand- und Fingerbereich zu schaffen. Die erfindungsgemäße Lösung besteht in den Merkmalen des Anspruchs 1 und vorzugsweise derjenigen der Unteransprüche.

Es ist ein Fixateur mit mindestens einer Fixateurstange und mit von dieser gehaltenen, zwischen ihnen parallel zueinander laufenden Kirschnerdrähten bekannt (WO88/05288), bei dem die aus weichem Material hergestellten biegbaren Fixateurstangen beidseitig eines frakturierten Knochens angeordnet sind. Zur Verbindung der Kirschnerdrähte mit den Fixateurstangen weisen letztere Öffnungen auf, in die die Kirschnerdrähte eingesteckt und mittels einer Spezialstange verpreßt werden. Nachteilig ist, daß zum einen Spezialwerkzeug erforderlich ist und zum anderen der Fixateur lediglich zur Therapie von Frakturen kleiner tubulärer Knochen geeignet ist.

Die erfindungsgemäß verwendeten Drähte können übliche Kirschner-Drähte sein. Sie sind leicht anzuwenden und können ohne Schwierigkeiten und mit bekannten Mitteln in eine gewünschte Form gebracht werden, falls sie nicht in gerader Gestalt Verwendung finden sollen. Ihre Halterung unmittelbar zwischen je zwei Muttern auf einer Gewindestange ist ebenso einfach wie wirkungsvoll, platzsparend und leicht. Dies gilt um so mehr, als die Lage der Drähte in bezug auf die Gewindestangen bis zu einem gewissen Gerade bereits durch die Lage der sie aufnehmenden Queröffnungen in den Gewindestangen vorbestimmt ist. Wenn es sich bei den Queröffnungen um einfache Bohrungen handelt, deren Maß dem Durchmesser der Drähte angepaßt ist, ist ihre Richtung weitgehend festgelegt. Wenn es sich bei den Queröffnungen um Längsschlitze handelt, deren Dicke nicht wesentlich größer ist als die der Drähte, sind sie festgelegt auf die Längsebene der Gewindestangen.

Die Gewindestangen sind gegenüber der Handelsware lediglich durch Einbringen der Queröffnungen abgewandelt. Als Muttern können übliche Sechskantmuttern verwendet werden. Stattdessen können aber auch solche Verwendung finden, die an dem Ende, das mit dem zu klemmenden Draht zusammenwirkt, zur Bildung einer im Durchmesser kleineren Anlagefläche konisch zulaufen. Eine solche kleinere Anlagefläche hat den Vorteil, daß sie sich mit einem möglicherweise gewünschten, geneigten Verlauf der Drähte gegenüber der Gewindestangenrichtung besser verträgt als eine große Schraubenstirnfläche.

Die Drähte können glatt sein, insbesondere wenn sie beidseitig des zu fixierenden Knochens an einem Paar von Gewindestangen gehalten sind. Jedoch besteht auch die Möglichkeit, solche Drähte zu verwenden, die an ihrem mit dem Knochen zusammenwirkenden Ende ein Gewinde aufweisen, um auch in ihrer Längsrichtung eine Haltewirkung auf den vorzugsweise vollständig von ihnen durchdrungenen Knochen ausüben zu können. Die Spitzen der Drähte können bekannterweise angespitzt und/oder mit Bohrschneiden oder Bohrkanten versehen sein, um unmittelbar in den zu haltenden Knochen eingestoßen oder eingebohrt werden zu können.

Die Erfindung ermöglicht sehr unterschiedliche Drahtanordnungen, beispielsweise mit zwei parallelen Drähten und einem Diagonaldraht im Falle von Schrägbrüchen oder mit zwei Paaren von Paralleldrähten im Falle eines Querbruchs, mit mehreren schräg angeordneten Gewindedrähten im Falle von gelenknahen Brüchen, sowie schließlich mit Fixierung durch eine oder mehrere Gewindestangen. Die unterschiedlichen Drahtanordnungen haben den Vorteil, daß es auch bei unterschiedlicher Lage des Bruchspalts wenigstens eine Drahtanordnung gibt, bei der ein Draht den Bruchspalt durchdringt.

Wegen der Einfachheit des Prinzips und seiner Anwendung kann das Anlegen des Fixateurs oftmals ohne die Voraussetzungen eines Operationssaals, beispielsweise im Notfallraum, durchgeführt werden, wobei allerdings Röntgenkontrolle zur Verfügung stehen sollte.

Die Erfindung wird im folgenden näher unter Bezugnahme auf die Zeichnung erläutert, die vorteilhafte Ausführungsbeispiele veranschaulicht. Darin zeigen:
- Fig. 1 bis 4: Anordnungsbeispiele an unterschiedlichen Knochen und
- Fig. 5 bis 10: Beispiele für verschiedene Ausführungen der Gewindestange und
- Fig. 11: eine Gewindestangenanordnung mit zwei unterschiedlichen Muttern.

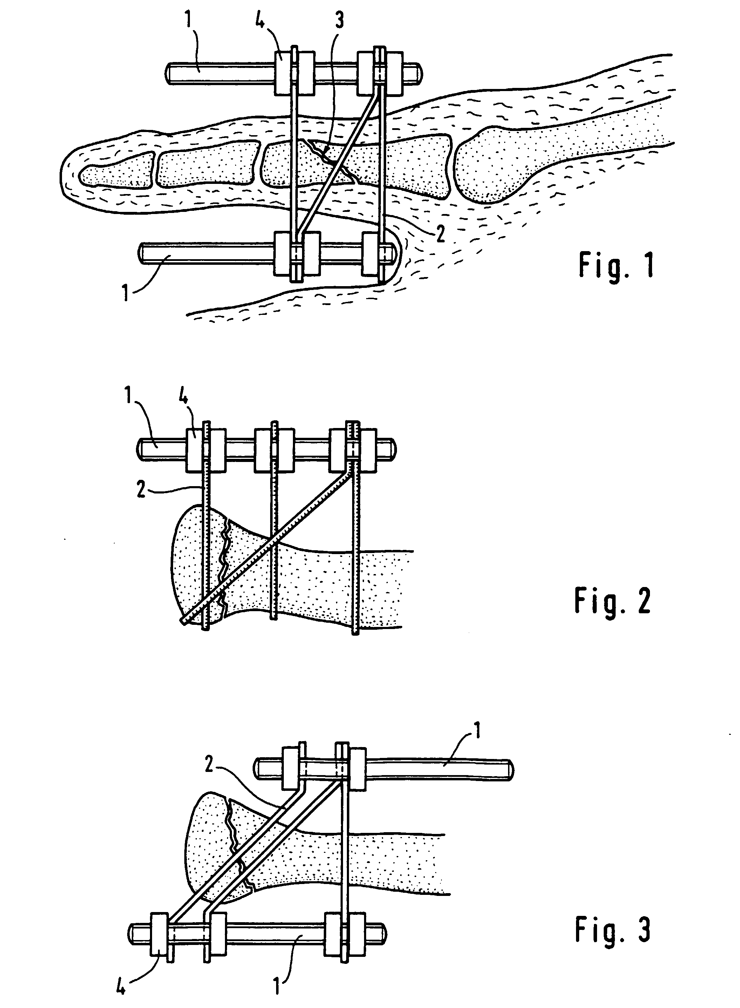

Fig. 1 zeigt die Anwendung des Fixateurs bei einem Schrägbruch des Kleinfinger-Grundgliedknochens. Der Fixateur besteht in diesem Fall aus zwei beiderseits des Fingers angeordneten Gewindestangen 1 und drei Drähten 2. Zunächst wird ein Kirschnerdraht 2 schräg durch den Bruch 3 gebohrt, der der vorläufigen Fixierung dient. Dann werden zwei Kirschner-drähte lotrecht zur Knochenachse eingebohrt, der eine proximal und der andere distal von der Bruchstelle. Dabei achtet man darauf, daß sämtliche Drähte in derselben Ebene liegen und die beide zuletzt eingebohrten Drähte im ausgerichteten Zustand des Knochens zueinander parallel sind. Die Drähte werden dann durch Schlitze in den Gewindestangen 1 geführt und mittels der Muttern 4 befestigt. Der schräg verlaufende Draht kann vorher an den die Gewindestangen durchdringenden Enden gebogen werden; unbedingt erforderlich ist dies jedoch im allgemeinen nicht, da die Muttern ohnehin nur mit mäßiger Kraft angezogen zu werden brauchen. Starker Anzug könnte ihre Position in ungewünschter Weise beeinflussen. Die überstehenden Enden der Drähte werden abgeschnitten. Nachdem die Anordnung mittels der Muttern 4 vorläufig fixiert wurde, kann ggf. nach Röntgenkontrolle eine Korrektur stattfinden.

Handelt es sich um einen Querbruch, so verwendet man stattdessen zwei Paare von parallelen Drähten, von denen jeweils ein Paar distal und ein paar proximal der Bruchstelle eingebohrt wird.

Man erkennt, daß die querverlaufenden Drähte die Strecksehne nicht beeinträchtigen. Ferner ist Gelenkbewegung weiterhin möglich, wodurch einer Versteifung vorgebeugt wird. Eine offene Operation ist nicht erforderlich. Es genügt lokale Betäubung.

Im Fall der Fig. 2 handelt es sich um einen gelenknahen Bruch eines Fingerknochens oder eines äußeren Mittelhandknochens, der nur einseitig zugänglich ist. Der Fixateur wird daher mit nur einer einseitig angeordneten Gewindestange 1 aufgebaut, wobei Drähte verwendet werden, deren den Knochen gänzlich durchdringender Abschnitt mit einem Gewinde versehen ist. Zwei zueinander parallele Drähte fixieren das proximale Fragment; ein dazu paralleler Draht sowie ein schräg geführter Draht fixieren das distale Fragment. Die Befestigung der Drähte an der Gewindestange 1 erfolgt durch direkte Klemmung zwischen Muttern 4.

Fig. 3 zeigt das Beispiels eines gelenknahen Bruchs an einem beidseitig zugänglichen Knochen. Der Fixateur verwendet daher zwei paarig angeordnete Gewindestangen 1. Der Bruch wird mittels zwei parallel und schräg geführten Drähten fixiert, deren räumliche Stabilität sichergestellt wird durch einen im proximalen Fragment lotrecht eingebohrten Draht. Die Drähte werden in den Gewindestangen teils zwischen zwei Muttern, teils zwischen dem gestrichelt angedeuteten Ende eines Längsschlitzes und einer Mutter gehalten.

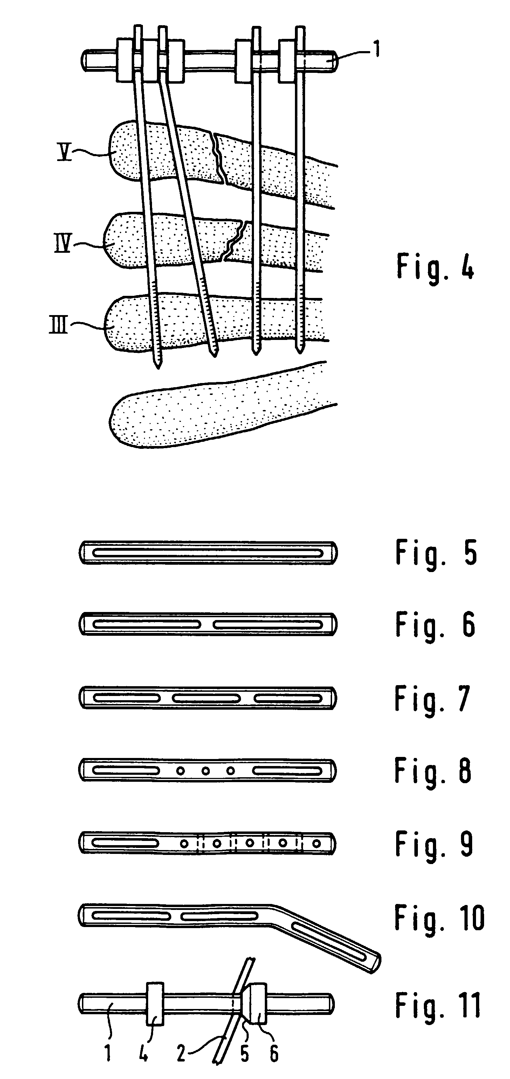

Fig. 4 veranschaulicht die Fixateuranordnung bei einem Bruch der Mittelhandknochen IV und V mittels Paaren von proximal und distal der Bruchstelle eingebohrten Drähte, deren freie Enden in dem Mittelhandknochen III mittels Gewinde verankert sind und deren äußere Enden von einer Gewindestange 1 gehalten sind.

Die Fig. 5 bis 10 zeigen Beispiele verschiedener Gewindestangen. Bei Fingerknochenfrakturen genügen in der Regel Stangen mit dem Gewinde M4 in Verbindung mit Drähten von 1,2 mm Durchmesser. Für größere Knochen oder bei Überbrückung größerer Längen, wie dies bei einer Fingergelenkversteifung notwendig sein kann, genügen in der Regel Gewindestangen M5 in Verbindung mit Kirschnerdrähten von 1,5 mm Durchmesser.

Die Fig. 5 bis 9 zeigen verschiedene Längsschlitz- und Bohrungsanordnungen. Bohrungen verwendet man, wenn die Richtung des Drahts gegenüber der Gewindestange unabhängig von der zu verwendenden Mutter festgelegt werden soll. Schlitze empfehlen sich dann, wenn Längskorrekturen notwendig sein können oder bei schräger Drahtanordnung. Mehrere voneinander durch Stege getrennte Längsschlitze (Fig. 6 und 7) haben den Vorteil größerer Stabilität und bieten in Gestalt der Stege Klemmanschläge an unterschiedlicher Position.

Die Beispiele zeigen die Anordnung der Drähte jeweils in nur einer Ebene. Es kann vorkommen, daß Drähte aus verschiedenen Richtungen in unterschiedlichen Ebenen eingebohrt werden müssen. In diesem Fall werden auf einer Seite des jeweiligen Glieds mehrere Gewindestangen verwendet, die etwa quer zu der Drahtanordnung miteinander verbunden werden können. Diesem Zweck dienen die in Fig. 9 dargestellten, quer zur Ebene des Längsschlites und der übrigen Bohrungen gestrichelt angedeuteten Bohrungen. Die Gewindestange gemäß Fig. 10 dient der Arthrodese und ist in dem gewünschten Winkel des zu versteifenden Gelenks gebogen.

Fig. 11 zeigt eine Gewindestange 1. Die darauf vorgesehene Normmutter 4 hat beiderseits verhältnismäßig großfläche Stirnflächen. Dies kann erwünscht sein, wenn Drähte lotrecht zur Gewindestangenrichtung zu befestigen sind. Wenn Drähte jedoch schräg verlaufen, kann eine Mutterstirnfläche 5 mit kleinerem Durchmesser zweckmäßiger sein, die an der Mutter 6 durch konisches Zulaufen derselben zu der Stirnfläche 5 ausgebildet ist.

Die Muttern können auch selbstsichernd ausgebildet sein, beispielsweise mit einem eingelegten Kunststoffring, wie dies an sich bekannt ist.

## Patentansprüche

1. Externer Fixateur, insbesondere für kleine Knochen wie Hand- und Fingerknochen, mit wenigstens einer Fixateurstange und einer Mehrzahl von wenigstens an einem Ende von der Fixateurstange gehaltenen, die zu fixierenden Knochen durchdringenden Drähten, dadurch gekennzeichnet, daß die Fixateurstange als Gewindestange (1) ausgebildet ist und daß wenigstens zwei der leicht biegbar ausgebildeten Drähte (2) in der sie und die Gewindestange (1) enthaltenden Ebene schräg zueinander verlaufen und die Gewindestange (1) mit die Drahtenden aufnehmenden Queröffnungen und Muttern (4) zum Festklemmen derselben ausgebildet ist.

2. Fixateur nach Anspruch 1, dadurch gekennzeichnet, daß die Muttern (4) die Drähte (2) unmittelbar klemmen.

3. Fixateur nach Anspruch 1 oder 2, dadurch gekennzeichnet, daß die Queröffnungen als Längsschlitze ausgebildet sind.

4. Fixateur nach einem der Ansprüche 1 bis 3, dadurch gekennzeichnet, daß die Queröffnungen als Bohrungen ausgebildet sind.

5. Fixateur nach einem der Ansprüche 1 bis 4, dadurch gekennzeichnet, daß die Queröffnungen zumindest in der Längsebene der Gewindestange (1) eng führend für die Drähte (2) ausgebildet sind.

6. Fixateur nach einem der Ansprüche 1 bis 5, dadurch gekennzeichnet, daß die Drähte (2) glatt sind.

7. Fixateur nach einem der Ansprüche 1 bis 6, dadurch gekennzeichnet, daß die Drähte (2) in ihrem mit dem Knochen zusammenwirkenden Abschnitt mit Gewinde versehen sind.

8. Fixateur nach einem der Ansprüche 1 bis 7, dadurch gekennzeichnet, daß die Spitzen der Drähte (2) selbstschneidend ausgebildet sind.

9. Fixateur nach einem der Ansprüche 1 bis 8, dadurch gekennzeichnet, daß die Drähte (2) an beiden Enden durch Gewindestangen miteinander verbunden sind.

10. Fixateur nach einem der Ansprüche 1 bis 9, dadurch gekennzeichnet, daß die Drähte (2) an einem Ende durch eine Gewindestange und am anderen Ende zur Verankerung in einem Knochen ausgebildet sind.

11. Fixateur nach einem der Ansprüche 1 bis 10, dadurch gekennzeichnet, daß wenigstens ein Draht (2) als Diagonaldraht ausgebildet ist.

12. Fixateur nach einem der Ansprüche 1 bis 11, dadurch gekennzeichnet, daß wenigstens zwei zueinander schräg verlaufende Drähte (2) zu einem gemeinsamen Befestigungspunkt zusammengeführt sind.

13. Fixateur nach einem der Ansprüche 1 bis 10, gekennzeichnet durch einen Drahtdurchmesser von nicht mehr als 2,5 mm, vorzugsweise nicht mehr als 1,8 mm.

14. Fixateur nach einem der Ansprüche 1 bis 11, insbesondere für Finger- oder Handknochen, dadurch gekennzeichnet, daß der Drahtdurchmesser zwischen 1 und 1,5 mm liegt.

15. Fixateur nach einem der Ansprüche 1 bis 12, gekennzeichnet durch einen Gewindestangen-Außendurchmesser im Bereich von 4 bis 5 mm.

16. Fixateur nach einem der Ansprüche 1 bis 13, dadurch gekennzeichnet, daß wenigstens eine Mutter (4) zu ihrer mit einem Draht zusammenwirkenden Stirnfläche (5) gegenüber ihrem sonstigen Durchmesser konisch zuläuft.

17. Fixateur nach einem der Ansprüche 1 bis 14, dadurch gekennzeichnet, daß wenigstens eine Mutter (4) selbstsichernd ausgebildet ist.

## Claims

1. An external fixator, in particular for small bones such as hand finger bones, having at least one fixator rod and a plurality of wires which are retained at at least one end by the fixator rod and which pass through the bones to be fixed, characterised in that the fixator rod is in the form of a threaded rod (1) and in that at least two of the wires (2), which are designed to be slightly flexible, extend obliquely relative to one another in the plane containing them and the threaded rod (1), and the threaded rod (1) is formed with transverse openings receiving the wire ends and with nuts (4) for the clamping thereof.

2. A fixator according to Claim 1, characterised in that the nuts (4) directly clamp the wires (2).

3. A fixator according to Claim 1 or 2, characterised in that the transverse openings are in the form of longitudinal slots.

4. A fixator according to any one of Claims 1 to 3, characterised in that the transverse openings are in the form of bores.

5. A fixator according to any one of Claims 1 to 4, characterised in that, at least in the longitudinal plane of the threaded rod (1), the transverse openings are designed to be closely guiding for the wires (2).

6. A fixator according to any one of Claims 1 to 5, characterised in that the wires (2) are smooth.

7. A fixator according to any one of Claims 1 to 6, characterised in that, in their portion co-operating with the bone, the wires (2) are provided with a screw-thread.

8. A fixator according to any one of Claims 1 to 7, characterised in that the tips of the wires (2) are designed to be self-cutting.

9. A fixator according to any one of Claims 1 to 8, characterised in that at both ends the wires (2) are joined together by threaded rods.

10. A fixator according to any one of Claims 1 to 9, characterised in that at one end the wires (2) are formed as threaded rods and at the other end are formed for anchoring in a bone.

11. A fixator according to any one of Claims 1 to 10, characterised in that at least one wire (2) is in the form of a diagonal wire.

12. A fixator according to any one of Claims 1 to 11, characterised in that at least two wires (2) extending obliquely relative to one another are brought together at a common fastening point.

13. A fixator according to any one of Claims 1 to 10, characterised by a wire diameter of not more than 2.5 mm, preferably not more than 1.8 mm.

14. A fixator according to any one of Claims 1 to 11, in particular for finger or hand bones, characterised in that the wire diameter is between 1 and 1.5 mm.

15. A fixator according to any one of Claims 1 to 12, characterised by a threaded rod outer diameter in the range of 4 to 5 mm.

16. A fixator according to any one of Claims 1 to 13, characterised in that at its end face (5) co-operating with a wire at least one nut (4) tapers conically with respect to its remaining diameter.

17. A fixator according to any one of Claims 1 to 14, characterised in that at least one nut (4) is designed to be self-locking.

## Revendications

1. Attelle externe, notamment pour petits os tels que les os de la main et des doigts, comportant au moins une tige et une pluralité de fils métalliques qui sont maintenus au moins à une extrémité de la tige de l'attelle et qui traversent les os à fixer, caractérisée en ce que la tige de l'attelle est réalisée sous la forme d'une tige filetée (1) et en ce qu'au moins deux des fils métalliques (2), réalisés légèrement flexibles, s'étendent obliquement l'un par rapport à l'autre dans le plan qui les contient et qui contient la tige filetée (1), et en ce que la tige filetée (1) présente des ouvertures transversales, qui reçoivent les extrémités des fils, et porte des écrous (4) pour serrer ces extrémités.

2. Attelle selon la revendication 1, caractérisée en ce que les écrous (4) serrent directement les fils métalliques (2).

3. Attelle selon la revendication 1 ou 2, caractérisée en ce que les ouvertures transversales sont réalisées sous la forme de fentes longitudinales.

4. Attelle selon l'une des revendications 1 à 3, caractérisée en ce que les ouvertures transversales sont réalisées sous la forme de perçages.

5. Attelle selon l'une des revendications 1 à 4, caractérisée en ce que les ouvertures transversales sont réalisées au moins dans le plan longitudinal de la tige filetée (1), de manière à guider étroitement les fils métalliques (2).

6. Attelle selon l'une des revendications 1 à 5, caractérisée en ce que les fils métalliques (2) sont lisses.

7. Attelle selon l'une des revendications 1 à 6, caractérisée en ce que les fils métalliques (2) sont pourvus d'un filetage dans leur partie coopérant avec l'os.

8. Attelle selon l'une des revendications 1 à 7, caractérisée en ce que les pointes des fils métalliques (2) sont autocoupantes.

9. Attelle selon l'une des revendications 1 à 8, caractérisée en ce que les fils métalliques (2) sont reliés entre eux à leurs deux extrémités par des tiges filetées.

10. Attelle selon l'une des revendications 1 à 9, caractérisée en ce que les fils métalliques (2) sont reliés à une extrémité par une tige filetée et sont réalisés à l'autre extrémité, en vue de leur ancrage dans un os.

11. Attelle selon l'une des revendications 1 à 10, caractérisée en ce qu'au moins un fil métallique (2) est réalisé sous la forme d'un fil diagonal.

12. Attelle selon l'une des revendications 1 à 11, caractérisée en ce qu'au moins deux fils métalliques (2), qui s'étendent obliquement l'un par rapport à l'autre, sont réunis en un point de fixation commun.

13. Attelle selon l'une des revendications 1 à 10, caractérisée par un diamètre des fils métalliques qui n'est pas supérieur à 2,5 mm, de préférence pas supérieur à 1,8 mm.

14. Attelle selon l'une des revendications 1 à Il, notamment pour les os des doigts ou de la main, caractérisé en ce que le diamètre des fils métalliques est compris entre 1 et 1,5 mm.

15. Attelle selon l'une des revendications 1 à 12, caractérisée par un diamètre extérieur de la tige filetée compris entre 4 et 5 mm.

16. Attelle selon l'une des revendications 1 à 13, caractérisée en ce qu'au moins un écrou (4) se termine coniquement, par rapport au reste de son diamètre, vers sa face frontale (5) coopérant avec un fil métallique.

17. Attelle selon l'une des revendications 1 à 14, caractérisée en ce qu'au moins un écrou (4) est autobloquant.
